# EUROPEAN PATENT SPECIFICATION

(11) **EP 2 356 461 B1**
(45) Date of publication and mention of the grant of the patent: **09.10.2013**
(21) Application number: 09748720.1
(22) Date of filing: 10.11.2009
(51) Int. Cl.: G01N 33/574

(54) **PACAP AS A MARKER FOR CANCER**
PACAP ALS KREBSMARKER
PACAP EN TANT QUE MARQUEUR POUR LE CANCER

(30) Priority: 12.11.2008 EP 08019731
(43) Date of publication of application: 17.08.2011
(73) Proprietor: Roche Diagnostics GmbH, 68305 Mannheim (DE); F. Hoffmann-La Roche AG, 4070 Basel (CH)
(72) Inventor: HAGMANN, Marie Luise, 82377 Penzberg (DE); KARL, Johann, 82380 Peissenberg (DE); RIEDLINGER, Julia, 85521 Ottobrunn (DE); ROESSLER, Markus, 82110 Germering (DE); TACKE, Michael, 80689 Muenchen (DE)
(74) Representative: Burger, Alexander
(86) International application number: PCT/EP2009/008006
(87) International publication number: WO 2010/054789

(56) References cited:
- WO-A-2006/060653
- WO-A-2007/112330
- SUZUKI M ET AL: "Gene expression profiling of human lymph node metastases and matched primary breast carcinomas: Clinical implications" MOLECULAR ONCOLOGY,, vol. 1, no. 2, 1 September 2007 (2007-09-01), pages 172-180, XP022823842 ISSN: 1574-7891 [retrieved on 2007-08-08]
- DUFFY M J: "CLINICAL USES OF TUMOR MARKERS: A CRITICAL REVIEW" CRITICAL REVIEWS IN CLINICAL LABORATORY SCIENCES, CRC PRESS, BACA RATON, FL, US, vol. 38, no. 3, 1 June 2001 (2001-06-01), pages 225-262, XP009042383 ISSN: 1040-8363 cited in the application

## Description

The present invention relates to a method aiding in the assessment of cancer. It discloses the use of the proapoptotic caspase adaptor protein (= PACAP) as a universal marker of different types of cancer. PACAP aids in the assessment of pulmonary or lung cancer (LC), particularly of non-small cell lung carcinoma (NSCLC), but also of other specific types of cancer. Such specific types of cancer are e.g. colon, bladder, cervix, ovary, endometrial, head and neck, breast, melanoma, pancreas, kidney, prostate, esophagus, stomach or bile duct cancer. Furthermore, the present invention especially relates to a method for assessing cancer from a liquid serum or plasma sample, derived from an individual by measuring PACAP in said sample. Measurement of PACAP can, e.g., be used in the early detection of cancer or in the surveillance of patients who undergo surgery.

Cancer remains a major public health challenge, despite progress in detection and therapy. Cancer cells are characterized by an atypical and unlimited growth that no longer is subject to the bodies growth control mechanisms. The dedifferentiation and neoplastic growth of tumor cells may lead to the production of cancer-associated marker proteins. Such cancer-associated proteins maybe found both in tissue and in the body fluids of an individual who carries cancer cells. Their levels usually are low at the early stages of the carcinogenic progress and increase during the diseases progression or in rare cases cancer-associated proteins are observed showing a decreased level in the course of disease progression. The identification of novel cancer-associated proteins and their sensitive detection is a big challenge, both to the technical experts as well as for public health systems. The most prevalent types of cancer are breast cancer (BC), lung cancer (LC) and colorectal cancer (CRC).

The most important therapeutic approaches for solid tumors are:
a) surgical resection of the tumor,
b) chemotherapy,
c) radiation therapy
d) treatment with biologicals, like anti-tumor antibodies or anti-angiogenic antibodies and
e) a combination of the above methods.

Surgical resection of the tumors is widely accepted as a first line treatment for early stage solid tumors. Most cancers, however, are detected only when they become symptomatic, i.e. when patients already are in a rather late stage of disease progression.

The staging of cancer is the classification of the disease in terms of extent, progression, and severity. It groups cancer patients so that generalizations can be made about prognosis and the choice of therapy.

Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, sixth edition, 2002). Once the TNM status is determined the patients are grouped into disease stages that are denoted by Roman numerals ranging form I to IV with IV being the most advanced disease stage. TNM staging and UICC disease stages correspond to each other as shown in the following Table taken from Sobin L.H. and Wittekind (eds.) supra.

### Interrelation of TNM staging and UICC disease stages

| **UICC disease stage** | **T staging** | **N staging** | **M staging** |
|---|---|---|---|
| Stage 0 | Tis | N0 | M0 |
| Stage I | T1, T2 | N0 | M0 |
| Stage IIA | T3 | N0 | M0 |
| Stage IIB | T4 | N0 | M0 |
| Stage IIIA | T1, T2 | N1 | M0 |
| Stage IIIB | T3, T4 | N1 | M0 |
| Stage IIIC | Any T | N2 | M0 |
| Stage IV | Any T | Any N | M1 |

What is especially important is that early diagnosis of cancer, e.g. of colorectal cancer (CRC) translates to a much better prognosis. In CRC malignant tumors of the colorectum arise from benign tumors, i.e. from adenoma. Therefore, best prognosis have those patients diagnosed at the adenoma stage. Patients diagnosed as early as in stage Tis, N0, M0 or T1-3; N0; M0, if treated properly have a more than 90% chance of survival 5 years after diagnosis as compared to a 5-years survival rate of only 10% for patients diagnosed when distant metastases are already present.

Current detection methods including imaging methods, such as X-ray or nuclear resonance imaging in theory might at least partially be appropriate for use as a general screening tool. However, they are very costly and not affordable to health care systems for a general and broad use in mass screenings of large numbers of subjects, particularly for subjects without any tumor symptoms.

It is an object of the present invention to provide a simple and cost-efficient procedure of tumor assessments, e.g. to identify individuals suspect of having cancer. For this purpose, a general tumor marker which is detectable in tissue samples or body fluids, e.g. blood or serum or plasma or a panel of such markers, would be desirable.

A number of serum tumor markers are already in clinical use. For example the soluble 30 kDa fragment of cytokeratin 19 (CYFRA 21-1), carcinoembryogenic antigen (CEA), neuron-specific enolase (NSE), and squamous cell carcinoma antigen (SCC) are the most prominent LC markers. However, none of them meets the criteria for sensitivity and specificity required for a screening tool (Thomas, L., Labor und Diagnose (2000), TH Books Verlagsgesellschaft, Frankfurt/Main, Germany).

In order to be of clinical utility, a new diagnostic marker as a single marker should be comparable to other markers known in the art, or better. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

Whole blood, serum or plasma are the most widely used sources of sample in clinical routine. The identification of an early tumor marker that would aid in the reliable cancer detection or provide early prognostic information could lead to a method that would greatly aid in the diagnosis and in the management of this disease. Therefore, an urgent clinical need exists to improve the in vitro assessment of cancer and in particular of LC. It is especially important to improve the early diagnosis of cancer, e.g. LC, since for patients diagnosed early on chances of survival are much higher as compared to those diagnosed at a progressed stage of disease.

The clinical utility of established biochemical markers in lung cancer has e.g. been reviewed by Duffy, M.J. (Crit. Rev. Clin. Lab. Sci. 38 (2001) 225-262) and shall be briefly discussed.

CYFRA 21-1 is currently regarded to be the best of the presently known tumor markers for lung cancer. Even though not organ-specific it is predominantly found in lung tissue. Sensitivity of CYFRA 21-1 for lung cancer is described to be between 46-61% at a specificity of 95% towards other benign lung diseases. Increased serum levels of CYFRA 21-1 are also associated with pronounced benign liver diseases, renal insufficiency and invasive bladder cancer. CYFRA 21-1 testing is recommended for postoperative therapy surveillance.

CEA belongs to the group of carcinofetal antigens, usually produced during embryogenesis. CEA is not organ-specific and predominantly used for monitoring of colorectal cancer. Besides malignancies, also several benign diseases such as cirrhosis, bronchitis, pancreatitis and autoimmune diseases are associated with increased CEA serum levels. At 95% specificity towards benign lung diseases its sensitivity for lung cancer is reported to be 29-44%. The primary use of CEA is in monitoring colon cancer, especially when the disease has metastasized. However, a variety of cancers can produce elevated levels of CEA, including breast cancer. A preferred use of CEA is therapy surveillance of lung cancer.

NSE is a tumor marker for SCLC. Generally, increased NSE serum levels are found in association with neuroectodermal and neuroendocrine tumors. Increased serum levels are also found in patients with benign lung diseases and cerebral diseases, such as meningitis or other inflammatory diseases of the brain, and traumatic injuries to the head. While sensitivity for SCLC at 95% specificity is reported to be 60-87%, performance of NSE testing for NSCLC is poor (7-25%). NSE is recommended for therapy surveillance of SCLC.

CA 19-9 (carbohydrate antigen 19-9), a sialylated Lewis (a) antigen) on a glycolipid is a tumor marker for gastrointestinal cancers. It occurs in fetal gastric, intestinal and pancreatic epithelia. Low concentrations can also be found in adult tissue in the liver, lungs, and pancreas. There is no correlation between tumor mass and the CA 19-9 assay values Therefore the determination of CA 19-9 cannot be used for the early detection of pancreatic carcinoma. As the mucin is excreted exclusively via the liver, even slight cholestasis can lead to clearly elevated CA 19-9 serum levels in some cases. The marker is mainly used as an aid in the monitoring of disease status in those patients having confirmed pancreatic cancer (sensitivity 70-87%). 3-7% of the population have the Lewis a-negative/b-negative blood group configuration and are unable to express the mucin with the reactive determinant CA 19-9. This must be taken into account when interpreting the findings.

CA 125 is found in a high percentage of non-mucinous ovarian tumors of epithelial origin and can be detected in serum. Ovarian carcinoma accounts for about 20% of gynecological tumors. Although the highest CA 125 values occur in patients suffering from ovarian carcinoma, clearly elevated values are also observed in malignancies of the endometrium, breast, gastrointestinal tract, and various other malignancies. Increased values are sometimes found in various benign gynecological diseases such as ovarian cysts, ovarian metaplasia, endometriosis, uterus myomatosus or cervicitis. Slight elevations of this marker may also occur in early pregnancy and in various benign diseases (e.g. acute and chronic pancreatitis, benign gastrointestinal diseases, renal insufficiency, autoimmune diseases and others). Markedly elevated levels have been found in benign liver diseases such as cirrhosis and hepatitis. Extreme elevations can occur in any kind of ascites due to malignant and benign diseases. Although CA 125 is a relatively unspecific marker, it is today the most important tumor marker for monitoring the therapy and progress of patients with serous ovarian carcinoma. A sensitivity of 69-79% is reported for 82-93% specificity.

SCC was originally identified in squamous cell CA of the cervix. The sensitivity of SCC for LC in general is low (18-27%). Therefore, SCC testing is regarded to be not suitable for screening. However, due to a higher sensitivity for squamous cell CA, a preferred use for SCC is therapy surveillance, even though CYFRA 21-1 generally performs better.

ProGRP is a tumor marker, useful in the detection and monitoring of SCLC. Increased serum levels are also found in patients with nonmalignant lung/pleural diseases, such as idiopathic pulmonary fibrosis or sarcoidosis. While sensitivity for proGRP in the field of SCLC (at 95% specificity) is reported to be 47-86%, the performance of proGRP testing in the field of NSCLC is poor because the sensitivity is reported as being below 10%.

CA 15-3 is usually increased in patients with advanced breast cancer. CA 15-3 levels are rarely elevated in women with early stage breast cancer (Duffy, M.J., Critical Reviews in Clinical Laboratory Sciences 38 (2001) 225-262). Cancers of the ovary, lung and prostate may also raise CA 15-3 levels. Elevated levels of CA 15-3 may be associated with non-cancerous conditions, such as benign breast or ovary disease, endometriosis, pelvic inflammatory disease, and hepatitis. Pregnancy and lactation can also cause CA 15-3 levels to raise (National Cancer Institute, Cancer Facts, Fact Sheet 5.18 (1998) 1-5).

CA 27-29 is found, similar to the CA 15-3 antigen, in the blood of most breast cancer patients. CA 27-29 levels may be used in conjunction with other procedures (such as mammograms and measurements of other tumor marker levels) to check for recurrence in women previously treated for stage II and stage III breast cancer. CA 27-29 levels can also be elevated by cancers of the colon, stomach, kidney, lung, ovary, pancreas, uterus, and liver. First trimester pregnancy, endometriosis, ovarian cysts, benign breast disease, kidney disease, and liver disease are noncancerous conditions that can also elevate CA 27-29 levels.

AFP (alpha-fetoprotein) is normally produced by a developing fetus. AFP levels begin to decrease soon after birth and are usually undetectable in the blood of healthy adults (except during pregnancy). An elevated level of AFP strongly suggests the presence of either primary liver cancer or germ cell cancer (cancer that begins in the cells that give rise to eggs or sperm) of the ovary or testicle. Only rarely do patients with other types of cancer (such as stomach cancer) have elevated levels of AFP. Noncancerous conditions that can cause elevated AFP levels include benign liver conditions, such as cirrhosis or hepatitis; ataxia telangiectasia; Wiscott-Aldrich syndrome; and pregnancy.

PSA (prostate-specific antigen) was used to a large extent as a reliable prognosis marker for the treatment of patients with prostate cancer (Catalona, W.J. et al., N. Engl. J. Med. 324 (1991) 1156-1161; Osterling, J.E., J. Urol. 145 (1991) 907-923). As a tumor marker it has the considerable advantage that it is not detectable in the blood of healthy men, or, if it is, only in very low concentrations. Highly elevated PSA values are generally measured in advanced stages of the disease. PSA belongs to a group of proteins/enzymes known as "kallikreins". The human kallirein family is composed of three members, described as hK1, hK2 and hK3 (PSA) (Clements, J.A., Endocr. Rev. 10 (1989) 393-419; Carbini, L.A. et al., J. Hypertens. 11 (1993) 893-898). The prostate-specific antigen (PSA), or hK3, is a glycoprotein with a molecular weight of approx. 29 kDa. It is formed in the prostatic epithelial cells and is a component of seminal fluid. PSA has the enzymatic activity of a neutral serin protease. Its main function is to cleave seminogelins I and II and fibronectin, which, as essential components of ejaculate, block the mobility of sperm. By hydrolyzing these proteins, PSA brings about the liquification of the seminal coagulum, which allows the mobility of sperm.

Ferritin is a macromolecule with a molecular weight of at least 440 kD (depending on the iron content) and consists of a protein shell (apoferritin) of 24 subunits and an iron core containing an average of approx. 2500 Fe³⁺ ions (in liver and spleen ferritin) (Wick, M. et al., Ferritin in Iron Metabolism - Diagnosis of Anemias, second edition, Springer-Verlag (1995), ISBN 3-211-82525-8 and ISBN 0-387-82525-8). Ferritin tends to form oligomers, and when it is present in excess in the cells of the storage organs there is a tendency for condensation to semicrystalline hemosiderin to occur in the lysosomes.

At least 20 isoferritins can be distinguished with the aid of isoelectric focusing (Arosio, P. et al., Heterogeneity of ferritin II: Immunological aspects, In: Albertini A., Arosio P., Chiancone E., Drysdale J. (eds.), Ferritins and isoferritins as biochemical markers, Elsevier, Amsterdam (1984) pp. 33-47). This microheterogeneity is due to differences in the contents of the acidic H and weakly basic L subunits. The basic isoferritins are responsible for the long-term iron storage function, and are found mainly in the liver, spleen, and bone marrow (Kaltwasser, J.P. et al., Serumferritin: Methodische und Klinische Aspekte, Springer Verlag (1980)).

Acidic isoferritins are found mainly in the myocardium, placenta, and tumor tissue. They have a lower iron content and presumably function as intermediaries for the transfer of iron in various syntheses (Morikawa, K. et al., Leuk. Lymphoma 18 (1995) 429-433; Borch-Iohnson, B., Analyst 120 (1995) 891-903; Cook, J. et al., Adv. Exp. Med. Biol. 356 (1994) 219-228).

The determination of ferritin is a suitable method for ascertaining the iron metabolism situation. Determination of ferritin at the beginning of therapy provides a representative measure of the body's iron reserves. Clinically, a threshold value of 20 µg/L (ng/mL) has proved useful in the detection of prelatent iron deficiency. This value provides a reliable indication of exhaustion of the iron reserves that can be mobilized for hemoglobin synthesis. When the ferritin level is elevated and the possibility of a distribution disorder can be ruled out, this is a manifestation of iron overloading in the body. 400 µg/L (ng/mL) ferritin is used as the threshold value. Elevated ferritin values are also encountered with the following tumors: acute leukemia, Hodgkin's disease and carcinoma of the lung, liver and prostate. The determination of ferritin has proved to be of value in liver metastasis. Studies indicate that 76% of all patients with liver metastasis have ferritin values above 400 µg/L (ng/mL). Reasons for the elevated values could be cell necrosis, blocked erythropoiesis or increased synthesis in tumor tissue.

On the other hand it was described as characteristic, that serum Ferritin levels did not increase in gastrointestinal cancer (Niitsu, Y. et al., Rinsho Ketsueki 21 (1980) 1135-1143). Furthermore, mean serum ferritin levels have been reported to be significantly lower in patients with advanced colorectal cancer than in controls (Kishida, T. et al., J. Gastroenterol. 29 (1994) 19-23. The decrease of serum Ferritin levels is caused by continous bleeding and this blood loss is related to the size and site of the tumor (Li, F. et al., J. Gastroenterol. 34 (1999) 195-199).

With respect to marker profiles and aiming at improved diagnosis of lung cancer, a method was published (Schneider, J. et al., Int. J. Clin. Oncol. 7 (2002) 145-151) using fuzzy logic based classification algorithms to combine serum levels of CYFRA 21-1, NSE and C-reactive protein (CRP) which is a general inflammation marker. The authors report a sensitivity of 92% at a specificity of 95%. However in this study, for example the sensitivity of CYFRA 21-1 as a single tumor marker is reported to be at 72% at a specificity of 95%, which is significantly higher than in many other reported studies. Duffy, M.J., in Crit. Rev. Clin. Lab. Sci. 38 (2001) 225-262 report a sensitivity of between 46% and 61%. This unusual high performance achieved by Schneider et al., raises some doubts and might be due to several facts. Firstly, the collective of control patients seems to be younger than the patients collective, i.e. the groups are not well age-matched, and the patient collective comprises many late stages. Secondly and even more critical, the performance of the algorithm is checked on the samples of the training set which were used for the determination of the fuzzy logic qualifiers. Hence, these qualifiers are strictly speaking "tailor-made" for this set and not applied to an independent validation set. Under normal circumstances, is has to be expected that the same algorithm applied to a larger, independent, and well balanced validation set will lead to a significantly reduced overall performance.

It was the task of the present invention to investigate whether a biochemical marker can be identified which may be used in assessing cancer disease. In particular, the inventors of the present invention investigated whether a biochemical marker could be identified for the assessment of different types of cancer, such as lung, colon, bladder, cervix, ovary, endometrial, head and neck, breast, melanoma, pancreas, kidney, prostate, esophagus, stomach and/or bile duct cancer in tissue samples or body fluids.

Surprisingly, it has been found that use of the PACAP protein as biomarker can at least partially overcome some of the problems of the markers presently known in the state of the art.

The present invention relates to a method for assessing cancer in vitro comprising measuring in a serum or plasma sample the concentration of a PACAP protein and/or fragments thereof and using the measurement results, particularly the concentration determined in the assessment of cancer.

Surprisingly, it was found that an increased concentration of a PACAP protein and/or fragments thereof in the test sample is associated with the occurrence of cancer. It could be shown that PACAP is a marker which is not specific for a single type of cancer, but a marker for different types of cancer, i.e. it appears to be a general tumor marker. Since PACAP appears to be rather specific for tumorgenic processes, as such the novel tumor marker PACAP has great potential to be of clinical utility with various classes of tumor types.

The method of the present invention is suitable for the assessment of many different types of cancer. Increased concentrations of PACAP protein and/or fragments thereof in a sample as compared to normal controls have been found for example in a specific type of cancer like lung, colon, bladder, cervix, ovary, endometrial, head and neck, breast, melanoma, pancreas, kidney, prostate, esophagus, stomach or bile duct cancer, respectively.

According to a preferred embodiment of the invention, the concentration of PACAP protein and/or fragments thereof is measured in a sample in order to assess in vitro a specific type of cancer, selected from the group consisting of lung, colon, bladder, cervix, ovary, endometrial, head and neck, breast, melanoma, pancreas, kidney, prostate, esophagus, stomach or bile duct cancer.

According to another preferred embodiment of the invention, the concentration of PACAP protein and/or fragments thereof is measured in a sample in order to assess cancer, such as lung, colon, bladder, cervix, ovary, endometrial, melanoma, or kidney cancer in vitro.

According to another preferred embodiment of the invention, the concentration of PACAP protein and/or fragments thereof is measured in a sample in order to assess cancer, such as lung, colon, bladder, cervix, ovary or endometrial cancer in vitro.

According to another preferred embodiment of the invention, the concentration of PACAP protein and/or fragments thereof is measured in a sample in order to assess cancer, such as lung cancer (LC) or colorectal cancer (CRC) in vitro.

According to another preferred embodiment of the invention, the concentration of PACAP protein and/or fragments thereof is measured in a sample in order to assess lung cancer (LC) in vitro.

One embodiment of the present invention refers to the mass screening of a population to distinguish between individuals which are probably free from cancer and individuals which might be classified as "suspect" cases. The latter group of individuals could then be subjected to further diagnostic procedures, e.g. by imaging methods or other suitable means.

A further embodiment of the present invention refers to an improvement of tumor marker panels which are suitable for the diagnosis of cancer in general or tumor marker panels which are suitable for the diagnosis of a specific tumor type, e.g. lung cancer.

The present invention is also directed to a method for assessing cancer in vitro by biochemical markers, comprising measuring in a sample the concentration of PACAP protein and/or fragments thereof and optionally of one or more other markers specific for cancer, and using the measurement results, particularly the concentrations, determined in the assessment of cancer. Preferred markers for use in combination with PACAP are, on the one hand, markers which are general tumor markers (i.e. markers which are not specific for a single tumor type) or, on the other hand, specific tumor markers (markers which are specific for a single tumor type). Preferred markers, e.g. for the assessment of cancer, such as lung cancer, are Cyfra 21-1, CEA, CA 19-9, SCC, CA 125, NSE, proGRP, CA 15-3, CA 27-29, AFP, PSA, Ferritin. These markers may be used individually each or in any combination together with PACAP.

If, according to this method of the invention, cancer is assessed, the one or more other marker of the respective cancer is preferably selected from the group consisting of Cyfra 21-1, CEA, CA 19-9, SCC, CA 125, NSE, proGRP, CA 15-3, CA 27-29, AFP, PSA, Ferritin.

Hence, the present invention, in a preferred embodiment, also relates to the use of a marker panel comprising at least the marker PACAP and at least one other tumor marker, e.g. of Cyfra 21-1, CEA, CA 19-9, SCC, CA 125, NSE, proGRP, CA 15-3, CA 27-29, AFP, PSA, Ferritin, in the assessment of cancer, e.g. lung and/or colon cancer.

Preferably, the present invention is directed to a method for assessing cancer, such as lung cancer in vitro by biochemical markers, comprising measuring in a serum or plasma sample the concentration of PACAP protein and/or fragments thereof and of optionally one or more other cancer markers, e.g. one or more other markers of lung cancer and using the measurement results, particularly concentrations determined in the assessment of cancer. It is preferred that the one or more other marker is selected from the group consisting of Cyfra 21-1, CEA, CA 19-9, SCC, CA 125, NSE and/or proGRP.

The present invention, in a preferred embodiment, also relates to the use of a marker panel comprising at least PACAP and CYFRA 21-1 in the assessment of cancer, particularly LC, and more particularly NSCLC.

The present invention also relates to the use of a marker panel comprising at least PACAP and CEA in the assessment of cancer, particularly LC, and more particularly NSCLC.

The present invention also relates to the use of a marker panel comprising at least PACAP and CA 19-9 in the assessment of cancer, particularly LC, and more particularly NSCLC.

The present invention also relates to the use of a marker panel comprising at least PACAP and SCC in the assessment of cancer, particularly LC, and more particularly NSCLC.

The present invention also relates to the use of a marker panel comprising at least PACAP and CA 125 in the assessment of cancer, particularly LC, and more particularly NSCLC.

The present invention also relates to the use of a marker panel comprising at least PACAP and NSE in the assessment of cancer, particularly LC, and more particularly NSCLC.

The present invention also relates to the use of a marker panel comprising at least PACAP and proGRP in the assessment of cancer, particularly LC, and more particularly NSCLC.

The present invention also relates to the use of a PACAP protein and/or fragments thereof in the assessment of cancer, wherein an increased concentration of PACAP protein and/or fragments thereof in a serum or plasma sample is indicative for cancer.

The present invention also relates to the use of PACAP in the assessment of several specific types of cancer, particularly lung, colon, bladder, cervix, ovary, endometrial, head and neck, breast, melanoma, pancreas, kidney, prostate, esophagus, stomach or bile duct cancer.

The present invention also relates to the use of an antibody directed against a PACAP protein and/or fragments thereof in the assessment of cancer, wherein an increased concentration of PACAP protein and/or fragments thereof in a serum or plasma sample is indicative for cancer.

In a preferred embodiment the present invention relates to a method for assessing cancer in vitro comprising measuring in a sample the concentration of a) a PACAP protein and/or fragments thereof, b) optionally one or more other marker of cancer, and (c) using the measurement results of step (a) and optionally of step (b) in the assessment of cancer, wherein said sample is serum or plasma, and wherein an increased concentration of PACAP protein and/or fragments thereof as compared to normal controls is indicative for cancer.

The term "measurement" preferably comprises a qualitative, a semi-qualitative or a quantitative measurement of a PACAP protein and/or fragments thereof in a sample. In a preferred embodiment the measurement is a semi-quantitative measurement, i.e. it is determined whether the concentration of PACAP is above or below a cut-off value. As the skilled artisan will appreciate, in a Yes- (presence) or No- (absence) assay, the assay sensitivity is usually set to match the cut-off value. A cut-off value can for example be determined from the testing of a group of healthy individuals. Preferably the cut-off is set to result in a specificity of 90%, also preferred the cut-off is set to result in a specificity of 95%, or also preferred the cut-off is set to result in a specificity of 98%. A value above the cut-off value can for example be indicative for the presence of cancer. In particular a value for PACAP above the cut-off value can for example be indicative for the presence of lung, colon, bladder, cervix, ovary, endometrial, head and neck, breast, melanoma, pancreas, kidney, prostate, esophagus, stomach or bile duct cancer. In a further preferred embodiment the measurement of PACAP is a quantitative measurement. In further embodiments the concentration of PACAP is correlated to an underlying diagnostic question like e.g. stage of disease, disease progression, or response to therapy.

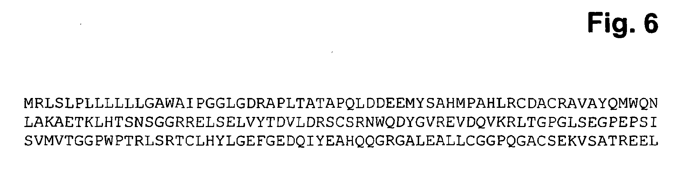

The proapoptotic caspase adaptor protein (PACAP, SwissProt database ID: PACAP_HUMAN, SwissProt database Accession No.: Q8WU39) is characterized by the sequence given in SEQ ID NO:1 (Figure 6). The PACAP molecule consists of 189 amino acids (= aa) and has a molecular weight of 20.7 kDa. Other synonyms for PACAP are FLJ32987, gxHOMSA73848, HSPC190, caspase-2 binding protein or hypothetical protein MGC29506. The corresponding human gene (coding sequence for PACAP) is located in chromosomal band 5q23-q31.

The human MGC29506 gene and the protein encoded therefrom was identified and characterized by Katoh and Katoh using EST information and bioinformatics (Katoh, M. and Katoh, M., Int. Journal of Oncology 23 (2003) 235-241). From the human MGC29506 gene three proteins are derived due to alternative splicing: the full length PACAP protein (SEQ ID NO: 1) with 189 aa as well as the splice variants Q8WU39-2 protein (SwissProt database ID: PACAP_HUMAN-S2, SwissProt database Accession No.: Q8WU39-2 with 123 aa; SEQ ID NO: 5) and the Q8WU39-3 protein (SwissProt database ID: PACAP_HUMAN-S3, SwissProt database Accession No.: Q8WU39-3 with 61 aa; SEQ ID NO: 6). The proteins Q8WU39 and Q8WU39-2 are identical in the N-terminal region comprising amino acids 1-59, but differ completely in the C-terminal region. Katoh and Katoh report that Q8WU39 (PACAP) and Q8WU39-2 (PACAP-2) are secreted-type proteins with the N-terminal signal peptide and six conserved cysteine residues. The Q8WU39 was shown to be the major expressed isoform of the MGC29506 gene. PACAP gene was found to be frequently down-regulated in intestinal-type gastric cancer (Katoh and Katoh). Because of this down-regulation they speculate that PACAP might be a candidate tumor suppressor gene implicated in intestinal-type gastric cancer. A determination of a PACAP protein in tissue samples or in a body fluid is not described. The document does not contain any data or indication that an elevated level of a PACAP protein might serve as a marker associated with cancer disease identification, prognosis and/or diagnosis.

Bonfoco et al. (J. Biol. Chem. 276 (2001) 29242-29250) report on the cloning, by a yeast two hybrid system, of the proapoptotic molecule PACAP. They identified the 123-amino acid protein PACAP-2 with a calculated molecular mass of 13.6 kDa. Using in vitro protein binding assays and Western blot analysis, Bonfoco et al. found that PACAP-2 interacts with caspase-2 and caspase-9, identifying the PACAP-2 protein as an apoptosis-triggering protein. The authors, however, do not describe any association of PACAP-2 with cancer assessment.

US 2006/0252057 discloses gene expression products or product combinations from a group of genes. The PACAP gene is mentioned as one of 331 genes. For several genes, mostly keratins, but not for PACAP, the differential expression was verified at the protein level by IHC. As the IHC analysis was made only for cancer tissue an altered expression compared to normal tissue was not shown.

WO 2006/121991 discloses the prognosis of breast cancer by quantification of the expression level of a group of genes of breast cancer patients as compared to a control group. Together with 67 other genes the PACAP gene, measured as mRNA, is described as being indicative of a good prognosis. Examples are given only for the determination of the corresponding mRNA levels of these genes by QPCR methodology. None of the examples shows the level of any protein neither in tissue nor in the circulation.

The same authors describe gene sets for the determination and prognosis of colon cancer in WO 2007/112330. The under-expression of the PACAP gene from a group with 8 other genes is disclosed to be indicative for a poor prognosis of colon cancer.

The examples of the last two patent applications use exclusively RNA detection methods for identification and quantification. The corresponding protein levels are not measured.

Hence none of the above documents of the art shows the measurement of PACAP protein in tissue samples or body fluids.

In addition it is well known that the correlation between the mRNA level and the protein level strongly varies (Kadota, K. et al., Genome Letters 2 (2003) 139-148; Greenbaum, D. et al., Genome Biology 4 (2003) 117). Thus the protein amount cannot be deduced from the corresponding mRNA levels. The conclusion that a deregulated mRNA level simultaneously leads to a deregulated protein level cannot be made.

WO 1997/38003 describes human hematopoietic-specific proteins (hHSP proteins). In a particular embodiment, WO 1997/38003 discloses the PACAP protein as human hematopoietic-specific protein and its use in the regulation of the differentiation and maturation of cells of the immune system and in the treatment and/or prevention of conditions characterized by an under-expression of hHSP proteins. A determination of the PACAP protein in tissue samples or in body fluids is not described. Further, the document does not contain any data that the PACAP protein might be a marker associated with cancer, and in particular with lung, breast and/or colon cancer.

WO 2006/060653 discloses methods and means (such as antibodies) for assessing lung cancer status comprising the steps of obtaining a biological sample from a lung cancer patient. Also the mRNA for markers such as PACAP and SCC is measured therein.

WO2007/112330 discloses that PACAP is measured in blood, faeces or other samples from a patient as a diagnostic marker for colon cancer. In particular, an underexpression of PACAP has been found to be indicative for a poor prognosis of colon cancer.

Suzuki M. et al., Molecular Oncology, vol. 1, no. 2 (2007) 172-180 have found that PACAP is overexpressed in lymph node metastases of breast carcinomas.

Hence, none of the above documents of the art suggests that a determination of PACAP in tissue samples and in body fluids would allow assessment of lung cancer.

Surprisingly, it was found in the present invention that a determination of the presence and/or amount of PACAP in a tissue sample and/or body fluid in the absence of morphological or histological information, particularly without determining subcellular localization, allows the assessment of cancer disease, e.g. of lung, breast, colon, prostate or kidney cancer disease, and in particular of lung cancer disease.

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising at least (the markers) PACAP and CYFRA 21-1 may optionally comprise one or more other marker.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing a patient's test sample. Examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be altered, e.g. during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=pI) or molecular weight (=MW), or both e.g., as a result of alternative mRNA or pre-mRNA processing. The amino acid sequence of a variant is to 95% or more identical to the corresponding marker sequence. In addition, or in the alternative a marker polypeptide or a variant thereof may carry a posttranslational modification. Non-limiting examples for posttranslational modifications are glycosylation, acylation, and/or phosphorylation.

### PACAP:

PACAP proteins, particularly soluble forms of PACAP proteins and/or fragments thereof, are detected in appropriate samples. Preferred samples are tissue samples or body fluids, such as blood, plasma, serum, urine, feces, bronchioalveolar lavage (BAL), epithelial lining fluid (ELF) or sputum. Preferably, the sample is derived from a human subject, e.g. a tumor patient or a person in risk of a tumor or a person suspected of having a tumor. Also preferred PACAP is detected in a serum or plasma sample.

In a preferred embodiment according to the present invention, the concentration of a PACAP protein and/or fragments thereof is determined. In one embodiment, the marker PACAP is specifically measured from a serum or plasma sample by use of a specific binding agent.

A specific binding agent is, e.g., a receptor for PACAP, a lectin binding to PACAP or an antibody to PACAP. A specific binding agent has at least an affinity of 10⁷ l/mol for its corresponding target molecule. The specific binding agent preferably has an affinity of 10⁸ l/mol or also preferred of 10⁹ l/mol for its target molecule. As the skilled artisan will appreciate the term specific is used to indicate that other biomolecules present in the sample do not significantly bind to the binding agent specific for PACAP. Preferably, the level of binding to a biomolecule other than the target molecule results in a binding affinity which is at most only 10% or less, only 5% or less only 2% or less or only 1% or less of the affinity to the target molecule, respectively. A preferred specific binding agent will fulfil both the above minimum criteria for affinity as well as for specificity.

A specific binding agent preferably is an antibody reactive with PACAP. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., supra, pages 108-115).

For the achievements as disclosed in the present invention polyclonal antibodies raised in rabbits may be used. However, clearly also polyclonal antibodies from different species, e.g., sheep or goat, as well as monoclonal antibodies can also be used. Since monoclonal antibodies can be produced in any amount required with constant properties, they represent ideal tools in development of an assay for clinical routine. The generation and the use of monoclonal antibodies to PACAP in a method according to the present invention, respectively, represent yet other preferred embodiments.

As the skilled artisan will appreciate now that PACAP has been identified as a marker which is useful in the assessment of cancer, preferably lung or colon cancer, various immunodiagnostic procedures may be used to reach a result comparable to the achievements of the present invention. For example, alternative strategies to generate antibodies may be used. Such strategies comprise amongst others the use of synthetic peptides, representing an epitope of PACAP for immunization. Alternatively, DNA immunization also known as DNA vaccination may be used.

For measurement the sample obtained from an individual is incubated with the specific binding agent for PACAP under conditions appropriate for formation of a binding agent PACAP complex. Such conditions need not be specified, since the skilled artisan without any inventive effort can easily identify such appropriate incubation conditions. The amount of binding agent PACAP complex is measured and the concentration of PACAP so determined is used in the assessment of cancer, preferably of lung cancer. As the skilled artisan will appreciate there are numerous methods to measure the amount of the specific binding agent PACAP complex all described in detail in relevant textbooks (cf., e.g., Tijssen P., supra, or Diamandis, E.P. and Christopoulos, T.K. (eds.), Immunoassay, Academic Press, Boston (1996)).

Preferably, PACAP is detected in a sandwich-type assay format. In such assays, a first specific binding agent is used to capture PACAP on the one side and a second specific binding agent, which is labeled to be directly or indirectly detectable, is used on the other side. The specific binding agents used in a sandwich-type assay format may be antibodies specifically directed against PACAP. The detection may be carried out by using different capturing and labeled antibodies, i.e. antibodies which recognize different epitopes on the PACAP protein.

A "marker of cancer" and in particular a "marker of lung cancer" in the sense of the present invention is any marker that if combined with the marker PACAP adds relevant information in the assessment of cancer in general or in the assessment of certain types of cancer, e.g. in the assessment of LC. The information is considered relevant, if the additive value, at a given specificity the sensitivity, or if at a given sensitivity the specificity, respectively, for the assessment of cancer can be improved by including said marker into a marker combination already comprising the marker PACAP. In the preferred embodiment of cancer assessment, the improvement in sensitivity or specificity, respectively, is statistically significant at a level of significance of p = .05, .02, .01 or lower. Preferably, the one or more other tumor marker is selected from the group consisting of Cyfra 21-1, CEA, CA 19-9, SCC, CA 125, NSE and/or proGRP.

The term "sample" as used herein refers to a biological sample obtained for the purpose of evaluation in vitro. In the methods of the present invention, the sample or patient sample preferably may comprise any body fluid or tissue sample. Preferred samples are whole blood, serum, plasma, bronchioalveolar lavage (BAL), epithelial lining fluid (ELF), urine or sputum, with plasma or serum being most preferred.

The term "tissue sample" and/or "tissue section" as used herein refers to a biological sample taken from a patient during surgery, therapeutic resections or a biopsy (e.g. incisional biopsy, excisional biopsy, core biopsy or needle aspiration biopsy) involving the removal of cells or tissues for the purpose of evaluation in vitro. When performing an analysis according to the present invention, the tissue sample material is used either directly or as a "tissue lysate". A "tissue sample" as used herein refers also to thin tissue slices usually accomplished through the use of a microtome. In any disclosed method embodiment involving a biological sample, such biological sample can be (but is not necessarily) mounted on a microscope slide, is a tissue section (such as a formalin-fixed and paraffin-embedded tissue section), and/or is a neoplastic tissue (such as, a lung cancer, colorectal cancer, head and neck cancer, gastric cancer, or glioblastoma).

A "tissue lysate", "cell lysate", "lysate", "lysed sample", "tissue extract" or "cell extract" as used herein refers to a sample and/or biological sample material comprising lysed tissue or cells, i.e. wherein the structural integrity of tissue or cells has been disrupted. To release the contents of cells or a tissue sample, the material is usually treated with enzymes and/or with chemicals to dissolve, degrade or disrupt the cellular walls and cellular membranes of such tissues or cells. The skilled artisan is fully familiar with appropriate methods for obtaining lysates. This process is encompassed by the term "lysis".

The term "assessing cancer" and in particular "assessing lung cancer" is used to indicate that the method according to the present invention will (alone or together with other markers or variables, e.g., the criteria set forth by the UICC (see above)) e.g., aid the physician to establish or confirm the absence or presence of cancer, in particular of LC, aid the physician in the prognosis, the detection of recurrence (follow-up of patients after surgery) and/or the monitoring of treatment, especially of chemotherapy.

As the skilled artisan will appreciate, any such assessment is made in vitro. The patient sample is discarded afterwards. The patient sample is solely used for the in vitro diagnostic method of the invention and the material of the patient sample is not transferred back into the patient's body. Typically, the sample is a liquid sample, e.g., whole blood, serum, or plasma.

Unless otherwise noted, technical terms are used according to conventional usage. Definitions of common terms in cell and molecular biology may be found in Lewin, B., Genes V, published by Oxford University Press (1994), ISBN 0-19-854287 9); Kendrew et al. (eds.), The Encyclopedia of Molecular Biology, published by Blackwell Science Ltd. (1994), ISBN 0-632-02182-9); and Meyers, R.A. (ed.), Molecular Biology and Biotechnology: a Comprehensive Desk Reference, published by VCH Publishers, Inc. (1995), ISBN 1-56081-569 8).

In a preferred embodiment the present invention relates to a method for assessing cancer, e.g. LC, in vitro by biochemical markers, comprising measuring in a serum or plasma sample the concentration of PACAP protein and/or fragments thereof and using the concentration determined in the assessment of cancer, e.g. LC.

In another preferred embodiment the present invention relates to a method for assessing LC in vitro by biochemical markers, comprising measuring in a serum or plasma sample the concentration of PACAP protein and/or fragments thereof and using the concentration determined in the assessment of LC.

The inventors of the present invention have surprisingly been able to detect an increased concentration of the marker PACAP in a significant percentage of samples derived from patients with cancer, in particular with lung, colon, bladder, cervix, ovary, endometrial, head and neck, breast, melanoma, pancreas, kidney, prostate, esophagus, stomach or bile duct cancer. Even more surprising they have been able to demonstrate that the increased concentration of PACAP protein and/or fragments thereof in such sample obtained from an individual can be used in the assessment of cancer, in particular of the above-mentioned types of cancer.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for many types of cancer, e.g. for LC. As the skilled artisan will appreciate, no biochemical marker is diagnostic with 100% specificity and at the same time 100% sensitivity for a given multifactorial disease, for example for LC. Rather, biochemical markers, e.g., CYFRA 21-1, CEA, CA 125, proGRP, SCC, or as shown here PACAP can be used to assess with a certain likelihood or predictive value e.g., the presence, absence, or the severity of a disease. Therefore in routine clinical diagnosis, generally various clinical symptoms and biological markers are considered together in the diagnosis, treatment and management of the underlying disease.

Biochemical markers can either be determined individually or in a preferred embodiment of the invention they can be measured simultaneously using a chip or a bead based array technology. The concentrations of the biomarkers are then either interpreted independently, e.g., using an individual cut-off for each marker, or they are combined for interpretation.

In a further preferred embodiment the assessment of cancer according to the present invention is performed in a method comprising measuring in a serum or plasma sample the concentration of a) a PACAP protein and/or fragment thereof, b) one or more other marker of cancer, and c) using the measurement result, e.g. the concentration determined in step (a) and step (b), respectively, in the assessment of cancer.

In the assessment of cancer the marker PACAP will be of advantage in one or more of the following aspects: screening; diagnostic aid; prognosis; monitoring of therapy such as chemotherapy, radiotherapy, and immunotherapy.

### Screening:

Screening is defined as the systematic application of a test to identify individuals e.g. at risk individuals, for indicators of a disease, e.g., the presence of cancer. Preferably the screening population is composed of individuals known to be at higher than average risk of cancer. For example, a screening population for lung cancer is composed of individuals known to be at higher than average risk of lung cancer, like smokers, ex-smokers, and uranium-, quartz- or asbestos-exposed workers.

In a preferred embodiment, a tissue sample or any body fluid such as plasma, serum, stool, urine, or sputum is used as a sample in the screening for cancer, e.g. lung cancer.

In another preferred embodiment of LC, sputum is used as a sample in the screening for lung cancer.

For many diseases, no single biochemical marker in the circulation will ever meet the sensitivity and specificity criteria required for screening purposes. This appears to be also true for cancer and in particular for lung cancer. It has to be expected that a marker panel comprising a plurality of markers will have to be used in cancer screening. The data established in the present invention indicate that the marker PACAP will form an integral part of a marker panel appropriate for screening purposes. The present invention therefore relates to the use of PACAP as one marker of a cancer marker panel, i.e. a marker panel comprising PACAP and one or more additional marker for cancer screening purposes. In particular, the present invention relates to the use of PACAP as one marker of a general cancer marker panel. Such marker panel comprises the marker PACAP and one or more additional markers, e.g. general cancer markers and/or markers for the above-mentioned type of cancer.

PACAP is also likely to contribute to marker panels for certain specific types of cancer, e.g. lung, colon, bladder, cervix, ovary, endometrial, head and neck, breast, melanoma, pancreas, kidney, prostate, esophagus, stomach or bile duct cancer.

A preferred type of cancer to be assessed with a marker panel comprising PACAP is lung cancer (LC).

The present data further indicate that certain combinations of markers will be advantageous in the screening for cancer. For example, with reference to the preferred embodiment of screening LC, the present invention also relates to the use of a marker panel comprising PACAP and CYFRA 21-1, or of a marker panel comprising PACAP and CEA, or of a marker panel comprising PACAP and CA 19-9, or of a marker panel comprising PACAP and SCC, or of a marker panel comprising PACAP and CA 125, or of a marker panel comprising PACAP and NSE, or of a marker panel comprising PACAP and proGRP, or of a marker panel comprising PACAP and two or more markers selected from the group consisting of Cyfra 21-1, CEA, CA 19-9, SCC, CA 125, NSE and/or proGRP.

### Diagnostic aid:

Markers may either aid the differential diagnosis of benign vs. malignant disease in a particular organ, help to distinguish between different histological types of a tumor, or to establish baseline marker values before surgery.

Today, important methods used in the detection of lung cancer are radiology and/or computed tomography (CT) scans. Small nodules, i.e. small regions of suspect tissue can be visualized by these methods. However, many of these nodules - more than 90% with CT - represent benign tissues changes, and only a minority of nodules represents cancerous tissue. Use of the marker PACAP may aid in the differentiation of benign versus malign disease.

In a preferred embodiment the marker PACAP is used in an immunohistological method in order to establish or confirm different histological types of lung, colon, bladder, cervix, ovary, endometrial, head and neck, breast, melanoma, pancreas, kidney, prostate, esophagus, stomach or bile duct, cancer, preferably LC.

Since PACAP as a single marker might be superior to other markers, e.g. in the case of LC to other markers, like Cyfra 21-1 or CEA it has to be expected that PACAP will be used as a diagnostic aid, especially by establishing a baseline value before surgery. The present invention thus also relates to the use of PACAP for establishing a baseline value before surgery for cancer.

### Prognosis:

Prognostic indicators can be defined as clinical, pathological, or biochemical features of cancer patients and their tumors that predict with a certain likelihood the disease outcome. Their main use is to help to rationally plan patient management, i.e. to avoid undertreatment of aggressive disease and overtreatment of indolent disease, respectively. Molina R. et al., Tumor Biol. 24 (2003) 209-218 evaluated the prognostic value of CEA, CA 125, CYFRA 21-1, SSC and NSE in NSCLC. In their study abnormal serum levels of the markers NSE, CEA, and LDH (lactate dehydrogenase) appeared to indicate shorter survival.

As PACAP alone significantly contributes to the differentiation of cancer patients, e.g. LC patients, from healthy controls, it has to be expected that it will aid in assessing the prognosis of patients suffering from cancer, preferably from LC. The level of preoperative PACAP will most likely be combined with one or more other marker for cancer and/or the TNM staging system. In a preferred embodiment PACAP is used in the prognosis of patients with LC.

### Monitoring of Chemotherapy:

Merle, P. et al., Int. J. of Biological Markers 19 (2004) 310-315 have evaluated CYFRA 21-1 serum level variations in patients with locally advanced NSCLC treated with induction chemotherapy. They conclude that early monitoring of CYFRA 21-1 serum levels may be a useful prognostic tool for tumor response and survival in stage III NSCLC patients. In addition, reports have described the use of CEA in monitoring the treatment of patients with LC (Fukasawa, T. et al., Gan to Kagaku Ryoho 13 (1986) 1862-1867) Most of these were retrospective, non-randomized and contained small numbers of patients. As in the case of the studies with CYFRA 21-1 the CEA studies suggested: a) that patients with a decrease in CEA levels while receiving chemotherapy generally had a better outcome than those patients whose CEA levels failed to decrease and (b) for almost all patients, increases in CEA levels were associated with disease progression.

It is expected that PACAP will be at least as good a marker for monitoring of chemotherapy as CYFRA 21-1 or CEA, respectively. The present invention therefore also relates to the use of PACAP in the monitoring of cancer patients and preferably of lung cancer (LC) patients under chemotherapy. In the monitoring of therapy in one preferred embodiment the measurements for PACAP and for at least one marker selected from the group consisting of Cyfra 21-1, CEA, CA 19-9, SCC, CA 125, NSE, and/or proGRP will be combined and used in the assessment of LC.

### Follow-up:

A large portion of LC patients who undergo surgical resection aimed at complete removal of cancerous tissue, later develop recurrent or metastatic disease (Wagner, H., Chest 117 (2000) 110S-118S; Buccheri, G. et al., Ann. Thorac. Surg. 75 (2003) 973-980). Most of these relapses occur within the first 2-3 years after surgery. Since recurrent/metastatic disease is invariably fatal if detected too late, considerable research has focused on cancer relapse at an early and thus potentially treatable stage.

Consequently, many cancer patients, e.g. LC patients undergo a postoperative surveillance program which frequently includes regular monitoring with CEA. Serial monitoring with CEA one year after surgical resection has been shown to detect an early postoperative recurrent/metastatic disease with a sensitivity of approximately 29 %, at a specificity of approximately 97 %, even in the absence of suspicious symptoms or signs (Buccheri, G. et al., Ann. Thorac. Surg. 75 (2003) 973-980). Thus, the follow-up of patients with LC after surgery is one of the most important fields of use for an appropriate biochemical marker. Due to the high sensitivity of PACAP in the LC patients investigated it is likely that PACAP alone or in combination with one or more other marker will be of great help in the follow-up of LC patients, especially in LC patients after surgery. The use of a marker panel comprising PACAP and one or more other marker of LC in the follow-up of LC patients represents a further preferred embodiment of the present invention.

The present invention in a preferred embodiment relates to the use of PACAP in the diagnostic field of cancer. Preferably PACAP is used in the assessment of lung, colon, bladder, cervix, ovary, endometrial, head and neck, breast, melanoma, pancreas, kidney, prostate, esophagus, stomach or bile duct cancer, respectively.

In yet a further preferred embodiment the present invention relates to the use of PACAP as a marker molecule for cancer, e.g. for cancer in general or for specific types of cancer, such as lung, colon, bladder, cervix, ovary, endometrial, head and neck, breast, melanoma, pancreas, kidney, prostate, esophagus, stomach or bile duct, cancer in combination with one or more further marker molecules for cancer.

The further marker molecules may be cancer-type unspecific general marker molecules and/or cancer-type specific marker molecules, e.g. marker molecules for lung or colon cancer. PACAP and the at least one further marker are used in the assessment of cancer, e.g. lung or colon cancer in a liquid sample obtained from an individual. Preferred selected other cancer markers with which the measurement of PACAP may be combined are Cyfra 21-1, CEA, CA 19-9, SCC, CA 125, NSE, proGRP, CA 15-3, CA 27-29, AFP, PSA, Ferritin. In particular, preferred selected other LC markers with which the measurement of PACAP may be combined are Cyfra 21-1, CEA, CA 19-9, SCC, CA 125, NSE and/or proGRP. Yet further preferred the marker panel used in the assessment of cancer, e.g. LC comprises PACAP and at least one other marker molecule selected from the group consisting of CYFRA 21-1 and CEA.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. the case when diagnosing an infectious disease, like AIDS.

Frequently, however, the combination of markers is evaluated. Preferably the values measured for markers of a marker panel, e.g. for PACAP and CYFRA 21-1, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of LC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics 12 (2003) 475-511; Friedman, J.H., J. of the American Statistical Association 84 (1989) 165-175; Hastie, T., et al., The Elements of Statistical Learning, Springer Series in Statistics (2001); Breiman, L., et al., Classification and regression trees, California: Wadsworth (1984); Breiman, L., Random Forests, Machine Learning 45 (2001) 5-32; Pepe, M.S., The Statistical Evaluation of Medical Tests for Classification and Prediction, Oxford Statistical Science Series 28 (2003); and Duda, R.O. et al., Pattern Classification, Wiley Interscience, 2nd edition (2001).

It is a preferred embodiment of the invention to use an optimized multivariate cut-off for the underlying combination of biological markers and to discriminate state A from state B, e.g. diseased from healthy. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup.

Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter e.g. is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Combining measurements of PACAP with other markers like CYFRA 21-1 or CEA, or with other markers of LC yet to be discovered, PACAP leads and will lead, respectively, to further improvements in assessment of LC.

In a preferred embodiment the present invention relates to a method for improving the diagnostic accuracy for cancer, e.g. LC versus healthy controls by measuring in a sample the concentration of at least PACAP and CYFRA 21-1, and optionally of CEA, CA 19-9, SCC, CA 125, NSE and/or proGRP, respectively and correlating the concentrations determined to the presence or absence of cancer, e.g. LC, the improvement resulting in more patients being correctly classified as suffering from cancer, e.g. LC versus healthy controls as compared to a classification based on any single marker investigated alone.

In a preferred method according to the present invention at least the concentration of the biomarkers PACAP and CYFRA 21-1, respectively, is determined and the marker combination is used in the assessment of cancer, e.g. LC.

In a further preferred method according to the present invention at least the concentration of the biomarkers PACAP and CEA, respectively, is determined and the marker combination is used in the assessment of cancer, e.g. LC.

In a further preferred method according to the present invention at least the concentration of the biomarkers PACAP and CA 19-9, respectively, is determined and the marker combination is used in the assessment of cancer, e.g. LC.

In a further preferred method according to the present invention at least the concentration of the biomarkers PACAP and SCC, respectively, is determined and the marker combination is used in the assessment of cancer, e.g. LC.

In a further preferred method according to the present invention at least the concentration of the biomarkers PACAP and CA 125, respectively, is determined and the marker combination is used in the assessment of cancer, e.g. LC.

In a further preferred method according to the present invention at least the concentration of the biomarkers PACAP and NSE, respectively, is determined and the marker combination is used in the assessment of cancer, e.g. LC.

In a further preferred method according to the present invention at least the concentration of the biomarkers PACAP and proGRP, respectively, is determined and the marker combination is used in the assessment of cancer, e.g. LC.

In yet a further preferred method according to the present invention at least the concentration of the biomarkers PACAP, CYFRA 21-1, and CEA, respectively, is determined and the marker combination is used in the assessment of cancer, e.g. LC.

In yet a further preferred method according to the present invention at least the concentration of the biomarkers PACAP, CYFRA 21-1, and CA 19-9, respectively, is determined and the marker combination is used in the assessment of cancer, e.g. LC.

In yet a further preferred method according to the present invention at least the concentration of the biomarkers PACAP, CYFRA 21-1, and SCC, respectively, is determined and the marker combination is used in the assessment of cancer, e.g. LC.

In yet a further preferred method according to the present invention at least the concentration of the biomarkers PACAP, CYFRA 21-1, and CA 125, respectively, is determined and the marker combination is used in the assessment of cancer, e.g. LC.

In yet a further preferred method according to the present invention at least the concentration of the biomarkers PACAP, CYFRA 21-1, and NSE, respectively, is determined and the marker combination is used in the assessment of cancer, e.g. LC.

In yet a further preferred method according to the present invention at least the concentration of the biomarkers PACAP, CYFRA 21-1, and proGRP, respectively, is determined and the marker combination is used in the assessment of cancer, e.g. LC.

The following examples, sequence listing and the figures are provided to aid the understanding of the present invention, the true scope of which is set forth in the appended claims.

### Description of the Figures

- **Figure 1**: Figure 1 shows an enlarged section of a typical 2D-gel, loaded with a tumor sample (panel B), and of a typical gel, loaded with a matched control sample (panel A). The circle indicates the position for the PACAP protein.
B = panel B, tumor sample;
A = panel A, control sample.
- **Figure 2**: Figure 2 shows the plot of the receiver operator characteristics (ROC-plot) for the assessment of 60 samples obtained from patients with LC as compared to 60 control samples obtained from 30 obviously healthy individuals and 30 apparently healthy smokers.
- **Figure 3**: Figure 3 shows a Western Blot analysis of lung cancer tissue lysates. 5 µg total protein of 20 lung cancer tissue lysates (10 adeno carcinoma and 10 squamous cell carcinoma) and matched control tissue lysates were analyzed as described in Example 5.
M = molecular weight marker;
T = tumour tissue lysate;
N = matched control tissue lysate;
arrows indicate the position of PACAP.
- **Figure 4**: Figure 4 shows a Western Blot analysis of breast, colon, prostate and kidney cancer tissue lysates. 5 µg total protein of respectively 20 cancer tissue lysates and matched control tissue lysates were analyzed as described in Example 6.
M = molecular weight marker;
T = tumour tissue lysate;
N = matched control tissue lysate;
Mamma = breast cancer tissue lysate;
Colon = colon cancer tissue lysate;
Prostate = prostate cancer tissue lysate;
Kidney = kidney cancer tissue lysate;
Bl. = bladder cancer tissue lysate; arrows indicate the position of PACAP.

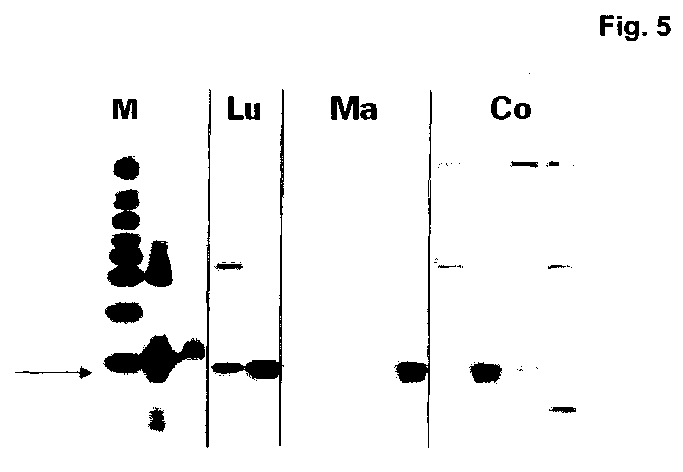

- **Figure 5**: Figure 5 shows a comparative Western Blot analysis of lung, breast and colon cancer tissue lysates. The cancer tissue lysates were analysed as described in Examples 5 and 6.
M = molecular weight marker;
Lu = lung cancer tissue lysate;
Ma = breast cancer tissue lysate;
Co = colon cancer tissue lysate;
arrows indicate the position of PACAP.
- **Figure 6**: Figure 6 shows the amino acid sequence of the PACAP protein (SEQ ID NO:1).

### Description of the Sequences:

- **SEQ ID NO: 1**: shows the sequence according to Figure 6. The human PACAP protein sequence (SwissProt database accession number: Q8WU39)
- **SEQ ID NO: 2**: shows a synthesized forward primer LC13Bfor-EcoRI
- **SEQ ID NO: 3**: shows a synthesized revers primer LC13Brev-HindIII
- **SEQ ID NO: 4**: shows a synthesized peptide extension
- **SEQ ID NO: 5**: shows a splice variant of the human PACAP gene: PACAP_HUMAN-S2 (Swissprot database accession number: Q8WU39-2)
- **SEQ ID NO: 6**: shows a splice variant of the human PACAP gene: PACAP_HUMAN-S3 (Swissprot database accession number: Q8WU39-3)

### Example 1

### Identification of PACAP as a marker for lung cancer

### Sources of tissue:

In order to identify tumor-specific proteins as diagnostic markers for lung cancer, analysis of two different kinds of tissue using proteomics methods is performed.

In total, tissue specimen from 20 patients suffering from lung cancer (10 adeno-CA and 10 squamous cell-CA) are analyzed. From each patient two different tissue types are collected from therapeutic resections: tumor tissue (>80% tumor) (T) and adjacent healthy tissue (N). The latter one serves as matched healthy control samples. Tissues are immediately snap frozen after resection and stored at -80°C before processing. Tumors are diagnosed by histopathological criteria.

### Tissue preparation:

0.8-1.2 g of frozen tissue are cut into small pieces, transferred to the chilled grinding jar of a mixer ball mill and completely frozen by liquid nitrogen. The tissue is pulverized in the ball mill, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton^{®} glass homogenizer (20 x loose fitting, 20 x tight fitting). The homogenate is subjected to centrifugation (10' at 5,000 x g), the supernatant is transferred to another vial and again subjected to centrifugation (15' at 20,000 x g). The resulting supernatant contains the soluble proteins and is used for further analysis.

### Depletion of albumin:

In order to deplete high abundant albumin from the prepared tissue lysates, the lysates were subjected to immunoaffinity chromatography, employing a monoclonal anti-human-albumin IgG coupled to CNBr-activated Sepharose 4B (GE Healthcare Europe GmbH, Munich, Germany). Tissue lysates were concentrated using an Amicon^{®} Ultra-15 device (Millipore GmbH, Schwalbach, Germany) following the instructions of the manufacturer. 1 ml of the concentrated lysates was injected onto the immunoaffinity column and subjected to chromatography with a flow of max. 1 ml/min. Running buffer was lysis buffer without Genapol X-080. The depleted flowthrough was collected and used for further analysis.

### Isoelectric focussing (IEF) and SDS-PAGE:

For IEF, 3 ml of the albumin-depleted, heparin-binding fraction were mixed with 12 ml sample buffer (7 M urea, 2 M thiourea, 2% CHAPS, 0.4% IPG buffer pH 4-7, 0.5% DTT) and incubated for 1 h. The samples were concentrated in an Amicon^{®} Ultra-15 device (Millipore GmbH, Schwalbach, Germany) and the protein concentration was determined using the Bio-Rad protein assay (Cat.No. 500-0006; Bio-Rad Laboratories GmbH, Munich, Germany) following the instructions of the supplier's manual. To a volume corresponding to 1.5 mg of protein sample buffer was added to a final volume of 350 µl. This solution was used to rehydrate IPG strips pH 4-7 (Amersham Biosciences, Freiburg, Germany) overnight. The IEF was performed using the following gradient protocol: 1.) 1 minute to 500 V; 2.) 2 h to 3500 V; 3.) 22 h at constant 3500V giving rise to 82 kVh. After IEF, strips were stored at -80 °C or directly used for SDS-PAGE.

Prior to SDS-PAGE the strips were incubated in equilibration buffer (6 M urea, 50 mM Tris/HCl, pH 8.8, 30% glycerol, 2% SDS), for reduction DDT (15 min, + 50 mg DTT/10 ml), and for alkylation IAA (15 min, + 235 mg iodacetamide/10 ml) was added. The strips were put on 12.5% polyacrylamide gels and subjected to electrophoresis at 1 W/gel for 1 h and thereafter at 17 W/gel. Subsequently, the gels were fixed (50% methanol, 10% acetate) and stained overnight with Novex^{®} Colloidal Blue Staining Kit (Invitrogen, Karlsruhe, Germany, Cat No. LC6025, 45-7101).

### Detection of PACAP protein as a potential marker for lung cancer:

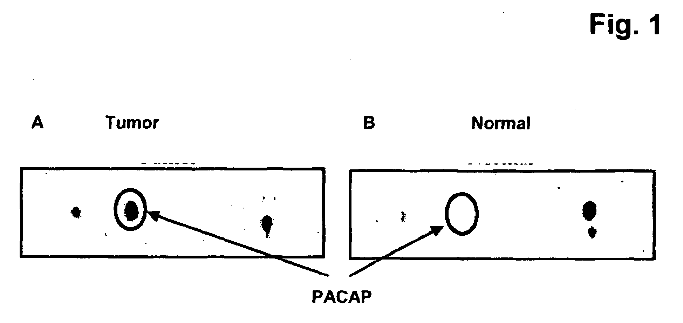

Each patient was analyzed separately by image analysis with the ProteomeWeaver^{®} software (Definiens AG, Munich, Germany). In addition, all spots of the gel were excised by a picking robot and the proteins present in the spots were identified by MALDI-TOF mass spectrometry (Ultraflex™ Tof/Tof, Bruker Daltonik GmbH, Bremen, Germany). For each patient, 3 gels from the tumor sample were compared with 3 gels from adjacent normal and analyzed for distinctive spots corresponding to differentially expressed proteins. By this means, PACAP protein was found to be specifically expressed or strongly overexpressed in tumor tissue. The PACAP protein spot was detected and identified in 2D-gels of 13 lung cancer patients but only in 4 control samples. Overexpression was similar in adeno carcinoma (AdCa) and squamous cell carcinoma (SCC) (see Table 1 and Figure 1). PACAP therefore qualified as a candidate marker for use in the diagnosis of lung cancer.

**Table 1:**

| **Overexpression of PACAP-protein in lung tumors** | | |
|---|---|---|
| | Protein detected in (number of samples) | |
| | Tumor | Control |
| Total | 13 | 4 |
| Adeno carcinoma (AdCa) | 7 | 1 |
| Squamous cell carcinoma (SCC) | 6 | 3 |

### Example 2

### Generation of antibodies to the lung cancer marker protein PACAP

Polyclonal antibody to the lung cancer marker protein PACAP is generated for further use of the antibody in the measurement of serum and plasma and blood levels of PACAP by immunodetection assays, e.g. Western Blotting and ELISA.

### Recombinant protein expression in E. coli:

In order to generate antibodies against PACAP, the recombinant antigen is produced in *E.coli:* Therefore, the PACAP coding region is PCR amplified from the full-length cDNA clone IRALp962G1939Q obtained from the German Resource Center for Genome Research (RZPD, Berlin, Germany) using the primers :
Forward primer LC13Bfor-*Eco*RI:
   5' ACGTACGT**GAATT**CATTAAAGAGGAGAAATTAACT
   **ATG**AGAGGATCG**CATCACCATCACCATCACATTGAAGGCCGT**agg_ctgtcactgc cactgctgc (SEQ ID NO: 2 / EcoRI - start codon underlined),
Reverse primer LC13Brev-HindIII:
   5' acgtacgtaa gctttcatta gagctcttct cttgtggctg (SEQ ID NO: 3).

The forward primer features (besides the *Eco*RI cloning and ribosomal binding sites) oligonucleotides coding for an N-terminal MRGSHHHHHHIEGR peptide extension (SEQ ID NO: 4) introduced in-frame to the PACAP protein. The *Eco*RI/*Hind*III digested PCR fragment is ligated into the corresponding pQE-30 (Qiagen, Hilden, Germany) vector fragment which is subsequently transformed into *E.coli* XL1-blue competent cells. After sequence analysis, the plasmid is transformed into *E.coli* BL21 competent cells for expression under the IPTG-inducible T5 promoter of the pQE vector series following the manufacturer's instructions.

For purification of the MRGSHHHHHHIEGR-PACAP fusion protein, 1 l of an over-night induced bacterial culture is pelleted by centrifugation and the cell pellet is lysed by resuspension in 100 mM sodium-phosphate buffer, pH 8.0, 7 M guanidium-hydrochloride, 5 mM imidazole, 20 mM thioglycerol. Insoluble material is pelleted by centrifugation and the supernatant is applied to Ni-nitrilotriacetic acid (Ni-NTA) metal-affinity chromatography: The column is washed with several bed volumes of lysis buffer followed by washes with a) 100 mM sodium-phosphate buffer, pH 8.0, 10 mM Tris-HCl, pH 8.0, 8 M urea, 20 mM thioglycerol; b) 100 mM sodium-phosphate buffer, pH 8.0, 0.5% sodium-dodecylsulfate (SDS), 20 mM thioglycerol; and c) 100 mM sodium-phosphate buffer, pH 8.0, 0.1% SDS, 20 mM thioglycerol. Finally, bound antigen is eluted using 100 mM sodium-phosphate buffer, pH 5.0, 0.1% SDS, 20 mM thioglycerol, under acid conditions, and stored in the same buffer at 4°C.

### Generation of polyclonal antibodies:

### a) Immunization

For immunization, a fresh emulsion of the protein solution (100 µg/ml protein PACAP) and complete Freund's adjuvant at the ratio of 1:1 is prepared. Each rabbit is immunized with 1 ml of the emulsion at days 1, 7, 14 and 30, 60 and 90. Blood is drawn and resulting anti-PACAP serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13 000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13 000 x g, 15 min, 4°C) and filtered (0.2 µm).

### Biotinylation of polyclonal rabbit IgG:

Polyclonal rabbit IgG is brought to 10 mg/ml in 10 mM NaH₂PO₄/NaOH, pH 7.5, 30 mM NaCl. Per ml IgG solution 50 µl Biotin-N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fractions containing biotinylated IgG are collected. Monoclonal antibodies are biotinylated according to the same procedure.

### Digoxigenylation of polyclonal rabbit IgG:

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex^{®} 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 3

### ELISA for the measurement of PACAP in human serum and plasma samples

For detection of PACAP in human serum or plasma, a sandwich ELISA is developed. For capture and detection of the antigen, aliquots of the anti- PACAP polyclonal antibody (see Example 2) are conjugated with biotin and digoxigenin, respectively.

Streptavidin-coated 96-well microtiter plates are incubated with 100 µl biotinylated anti-PACAP polyclonal antibody for 60 min at 10 µg/ml in 10 mM phosphate, pH 7.4, 1% BSA, 0,9% NaCl and 0.1% Tween 20. After incubation, plates are washed three times with 0.9% NaCl , 0.1% Tween 20. Wells are then incubated for 2 h with either a serial dilution of the recombinant protein (see Example 2) as standard antigen or with diluted plasma samples from patients. After binding of PACAP, plates are washed three times with 0.9% NaCl, 0.1% Tween 20. For specific detection of bound PACAP, wells are incubated with 100 µl of digoxygenylated anti- PACAP polyclonal antibody for 60 min at 10 µg/ml in 10 mM phosphate, pH 7.4, 1% BSA, 0,9% NaCl and 0.1% Tween 20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0,9% NaCl and 0.1% Tween 20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 4

### Clinical utility of PACAP in lung cancer

The clinical utility of PACAP is assessed by analyzing serum samples obtained from well characterized patient cohort. Two study populations differing in number and population of controls and patients are performed. The diagnostic value is evaluated by ROC-analysis and the determination of the sensitivity at 95 % specificity.

### First study

Samples derived from 60 well-characterized NSCLC patients (30 adeno-CA, 30 squamous cell CA) with the UICC classification given in Table 2 are used.

**Table 2:**

| **Study population** | |
|---|---|
| **Stage according to UICC** | **Number of samples** |
| UICC I/II | 24 |
| UICC III | 17 |
| UICC IV | 19 |
| obviously healthy blood donors | 30 |
| apparently healthy smokers | 30 |

The level of PACAP in the LC samples of Table 2 is evaluated in comparison to 60 control samples obtained from 30 healthy individuals and 30 apparently healthy smokers without any known malignant lung disease (= control cohort).

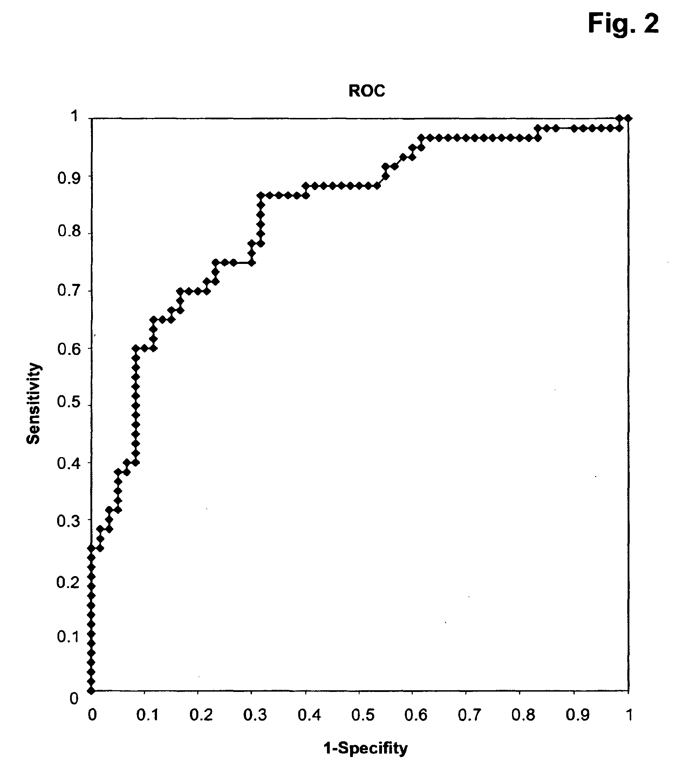

Discriminatory power for differentiating patients in the LC group from healthy individuals as measured by the area under the curve is found to be 82 % for LC vs. healthy controls (Figure 2).

At 95 % specificity the sensitivity for all LC samples was 37%, for adeno carcinomas 23% and for squamous cell carcinomas 50% respectively.

### Second study

A high number of clinically well characterized serum samples were collected in a multi center study (Table 3). In addition to samples from healthy persons and from apparently healthy smokers or ex-smokers the total control collective (233 samples) includes samples from individuals with an increased risk for lung cancer through a work-related exposure and from subjects with stages 0-II of chronic obstructive pulmonary disease (COPD).

In the age matched lung cancer group (Table 4) the most important histological types of NSCLC from all stages are represented as well as samples from SCLC patients. The AUC values of the ROC analysis for the NSCLC samples was 69 % and for the SCLC samples 68%. Values from 62-74% are obtained by grouping the NSCLC samples by UICC stages. From the different histology groups the large cell carcinoma samples discriminates best (77% AUC). Accordingly the sensitivity at 95% selectivity was highest for this histology type.

**Table 3:**

| **Control sample characteristics of the second study population:** | | | | |
|---|---|---|---|---|
| **Control collective** | | | **Number** | **Age (years)** |
| **Total** | | | **233** | **47.7 +/- 14.9** |
| **Healthy Non-Smoker or Ex-Smoker (<5 pack years)** | | | **50** | **41.4 +/- 9.4** |
| **Apparently Smoker/Ex-smoker** | | | **89** | **40.2 +/- 9.9** |
| | **COPD** | | **46** | **69.0 +/- 12.8** |
| | | Stage 0 | 5 | 56.4 +/- 12.9 |
| | | Stage I | 11 | 59.5 +/- 11.3 |
| | | Stage II | 30 | 66.3 +/- 12.9 |
| | **Work-related exposure** | | **48** | **53.0 +/- 15.9** |
| | | Asbestos | 11 | 56.6 +/- 14.9 |
| | | Dust | 10 | 34.6 +/- 7.4 |
| | | Radiation | 26 | 58.6 +/- 13.5 |
| | | Silicon | 1 | 50 |

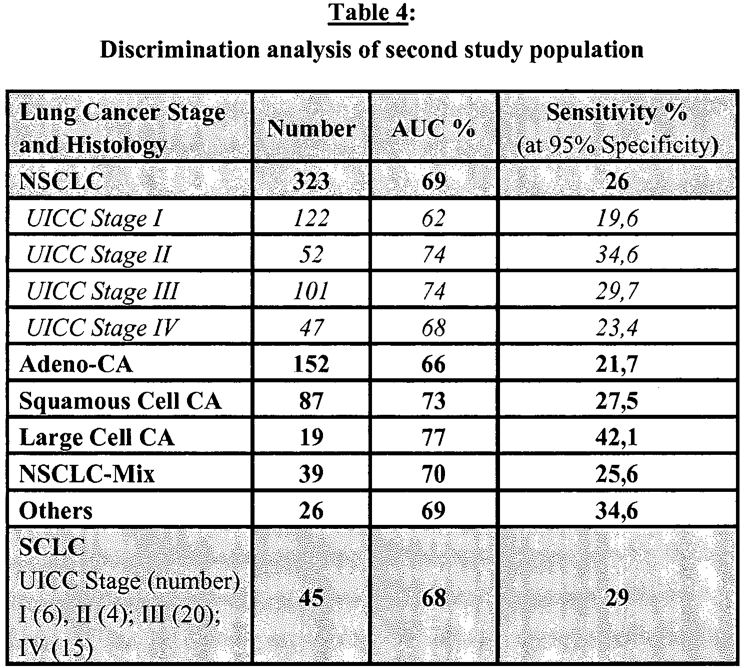

### Example 5

### Western Blotting for the detection of PACAP in human lung cancer tissue using polyclonal antibody as generated in Example 2

Tissue lysates from tumor samples and healthy control samples are prepared as described in Example 1, "Tissue preparation".

SDS-PAGE and Western-Blotting are carried out using reagents and equipment of Invitrogen, Karlsruhe, Germany. For each tissue sample tested, 15 µg of tissue lysate are diluted in reducing NuPAGE^{®} (Invitrogen) SDS sample buffer and heated for 10 min at 95°C. Samples are run on 4-12% NuPAGE^{®} gels (Tris-Glycine) in the MES running buffer system. The gel-separated protein mixture is blotted onto nitrocellulose membranes using the Invitrogen XCell II^{®} Blot Module (Invitrogen) and the NuPAGE^{®} transfer buffer system. The membranes are washed 3 times in PBS/0.05% Tween-20 and blocked with Roti^{®}-Block blocking buffer (A151.1; Carl Roth GmbH, Karlsruhe, Germany) for 2 h. The primary antibody, polyclonal rabbit anti-Q8WU39 serum (generation described in Example 2), is diluted 1:10,000 in Roti^{®}-Block blocking buffer and incubated with the membrane for 1 h. The membranes are washed 6 times in PBS/0.05% Tween-20. The specifically bound primary rabbit antibody is labeled with an POD-conjugated polyclonal sheep anti-rabbit IgG antibody, diluted to 10 mU/ml in 0.5 x Roti^{®}-Block blocking buffer. After incubation for 1 h, the membranes are washed 6 times in PBS/0.05% Tween-20. For detection of the bound POD-conjugated anti-rabbit antibody, the membrane is incubated with the Lumi-Light^{PLUS} Western Blotting Substrate (Order-No. 2015196, Roche Diagnostics GmbH, Mannheim, Germany) and exposed to an autoradiographic film.

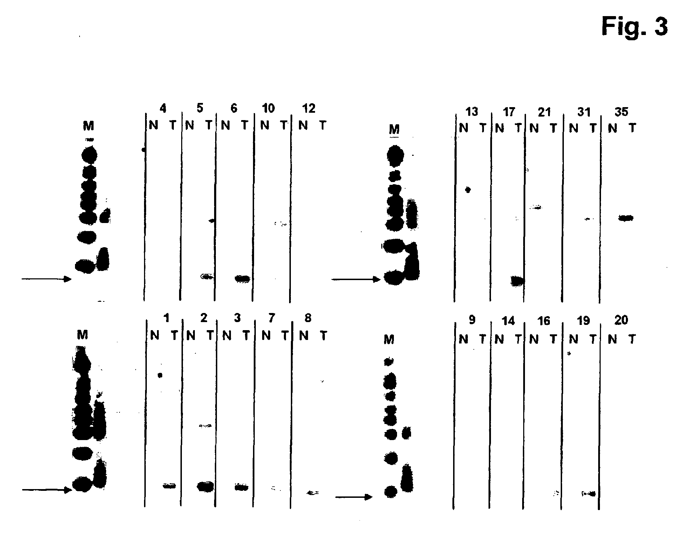

Signal intensity for PACAP was increased in 16 out of 20 tumor tissue lysates obtained from 20 different LC patients (Fig. 3). The expression level was >100 ng/mg. Thus, the increased abundance of PACAP in lung cancer tissue as detected by MALDI in Example 1 is confirmed by Western Blotting analyses.

### Example 6

### Western Blotting for the detection of PACAP in human breast and colon tissue using polyclonal antibody as generated in Example 2

Tissue lysates from tumor samples and healthy control samples were prepared as described in Example 1, "Tissue preparation".

SDS-PAGE and Western Blotting were carried out as described in Example 5.

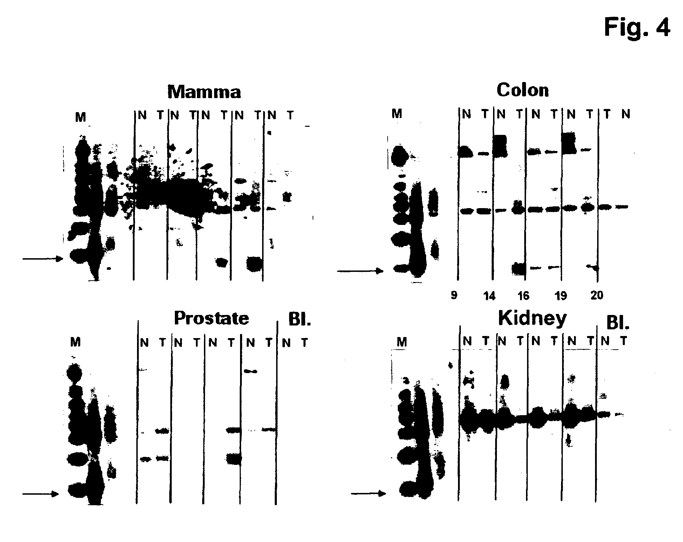

For both mamma and colon carcinoma, tumor and adjacent normal tissue of 5 patients were analyzed. Overexpression of the PACAP protein was found in 3 mamma cancers and 4 colon cancers compared to the normal tissue. The expression level varied within the samples and was a slightly lower in all mamma and colon cancers than in the lung cancers. The increased abundance of PACAP in mamma and colon cancer tissue was confirmed by Western Blotting analysis (Fig. 4 and 5).

## Claims

1. A method for assessing lung cancer in vitro comprising measuring in a sample the concentration of
(a) proapoptotic caspase adaptor protein (PACAP) and/or fragments thereof, and
(b) using the measurement result of step (a) in the assessment of cancer, wherein said sample is serum or plasma, and wherein an increased concentration of a PACAP protein and/or fragments thereof as compared to normal controls is indicative for lung cancer.

2. The method according to claim 1, wherein additionally one or more other marker of cancer is measured.

3. The method according to claim 2, further **characterized in that** said one or more other marker is selected from the group consisting of Cyfra 21-1, CEA, CA 19-9, SCC, CA 125, NSE and/or proGRP.

4. The method according any one of claims 1 to 3, further **characterized in that** the concentration is measured by an immunological method.

5. Use of PACAP protein and/or fragments thereof in the assessment of lung cancer, wherein an increased concentration of a PACAP protein and/or fragments thereof in a serum or plasma sample as compared to normal controls is indicative for lung cancer.

6. The use according to claim 5 in the assessment of lung cancer, particularly non small cell lung cancer (NSCLC).

7. The use of an antibody directed against a PACAP protein and/or fragments thereof in the assessment of lung cancer, wherein an increased concentration of a PACAP protein and/or fragments thereof in a serum or plasma sample as compared to normal controls is indicative for lung cancer.

8. The use of a marker panel comprising a PACAP protein and/or fragments thereof and one or more other marker for cancer in the assessment of lung cancer, wherein an increased concentration of a PACAP protein and/or fragments thereof in a serum or plasma sample as compared to normal controls is indicative for lung cancer.

9. The use of the marker panel according to claim 8, further **characterized in that** the one or more other marker is selected from the group consisting of Cyfra 21-1, CEA, CA 19-9, SCC, CA 125, NSE and/or proGRP.

10. The use of the marker panel according to claims 8 to 9 in the assessment of lung cancer, particularly NSCLC.

## Patentansprüche

1. Verfahren zur Beurteilung von Lungenkrebs in vitro, wobei man in einer Probe die Konzentration von
a. proapoptotischem Caspase-Adaptorprotein (PACAP) und/oder Fragmenten davon misst und
b. das Messergebnis aus Schritt (a) bei der Krebsbeurteilung verwendet, wobei es sich bei der Probe um Serum oder Plasma handelt und wobei eine erhöhte Konzentration eines PACAP-Proteins und/oder von Fragmenten davon im Vergleich zu Normalkontrollen ein Anzeichen für Lungenkrebs ist.

2. Verfahren nach Anspruch 1, wobei zusätzlich ein oder mehrere andere Krebsmarker gemessen wird bzw. werden.

3. Verfahren nach Anspruch 2, ferner **dadurch gekennzeichnet, dass** der eine bzw. die mehreren anderen Marker aus der aus Cyfra 21-1, CEA, CA 19-9, SCC, CA 125, NSE und/oder proGRP bestehenden Gruppe ausgewählt ist bzw. sind.

4. Verfahren nach einem der Ansprüche 1 bis 3, ferner **dadurch gekennzeichnet, dass** die Konzentration mit einem immunologischen Verfahren gemessen wird.

5. Verwendung eines PACAP-Proteins und/oder von Fragmenten davon bei der Beurteilung von Lungenkrebs, wobei eine erhöhte Konzentration eines PACAP-Proteins und/oder von Fragmenten davon in einer Serum- oder Plasmaprobe im Vergleich zu Normalkontrollen ein Anzeichen für Lungenkrebs ist.

6. Verwendung nach Anspruch 5 bei der Beurteilung von Lungenkrebs, insbesondere nichtkleinzelligem Lungenkrebs (NSCLC).

7. Verwendung eines gegen ein PACAP-Protein und/oder Fragmente davon gerichteten Antikörpers bei der Beurteilung von Lungenkrebs, wobei eine erhöhte Konzentration eines PACAP-Proteins und/oder von Fragmenten davon in einer Serum- oder Plasmaprobe im Vergleich zu Normalkontrollen ein Anzeichen für Lungenkrebs ist.

8. Verwendung einer ein PACAP-Protein und/oder Fragmente davon sowie einen oder mehrere andere Krebsmarker umfassenden Markergruppe bei der Beurteilung von Lungenkrebs, wobei eine erhöhte Konzentration eines PACAP-Proteins und/oder von Fragmenten davon in einer Serum- oder Plasmaprobe im Vergleich zu Normalkontrollen ein Anzeichen für Lungenkrebs ist.

9. Verwendung der Markergruppe nach Anspruch 8, ferner **dadurch gekennzeichnet, dass** der eine bzw. die mehreren anderen Marker aus der aus Cyfra 21-1, CEA, CA 19-9, SCC, CA 125, NSE und/oder proGRP bestehenden Gruppe ausgewählt ist bzw. sind.

10. Verwendung der Markergruppe nach Anspruch 8 bis 9 bei der Beurteilung von Lungenkrebs, insbesondere NSCLC.

## Revendications

1. Procédé pour l'évaluation d'un cancer du poumon in vitro comprenant la mesure dans un échantillon de la concentration de
(a) protéine adaptateur de la caspase pro-apoptotique (PACAP) et/ou des fragments de celle-ci, et
(b) l'utilisation du résultat de la mesure de l'étape (a) dans l'évaluation d'un cancer, dans lequel ledit échantillon est du sérum ou du plasma, et dans lequel une concentration accrue d'une protéine PACAP et/ou des fragments de celle-ci par rapport à des témoins normaux est indicatrice d'un cancer du poumon.

2. Procédé selon la revendication 1, dans lequel en outre un ou plusieurs autre(s) marqueur(s) du cancer est(sont) mesuré(s).

3. Procédé selon la revendication 2, **caractérisé en outre en ce que** ledit un ou plusieurs autre marqueur est choisi dans le groupe constitué par Cyfra 21-1, CEA, CA 19-9, SCC, CA 125, NSE et/ou proGRP.

4. Procédé selon l'une quelconque des revendications 1 à 3, **caractérisé en outre en ce que** la concentration est mesurée par une méthode immunologique.

5. Utilisation d'une protéine PACAP et/ou des fragments de celle-ci dans l'évaluation d'un cancer du poumon, dans laquelle une concentration accrue d'une protéine PACAP et/ou des fragments de celle-ci dans un échantillon de sérum ou de plasma par rapport à des témoins normaux est indicatrice d'un cancer du poumon.

6. Utilisation selon la revendication 5 dans l'évaluation d'un cancer du poumon, en particulier le cancer du poumon non à petites cellules (CPNPC).

7. Utilisation d'un anticorps dirigé contre une protéine PACAP et/ou les fragments de celle-ci dans l'évaluation d'un cancer du poumon, dans laquelle une concentration accrue d'une protéine PACAP et/ou des fragments de celle-ci dans un échantillon de sérum ou de plasma par rapport à des témoins normaux est indicatrice d'un cancer du poumon.

8. Utilisation d'un panel de marqueurs comprenant une protéine PACAP et/ou des fragments de celle-ci et un ou plusieurs autre(s) marqueur(s) du cancer dans l'évaluation d'un cancer du poumon, dans laquelle une concentration accrue d'une protéine PACAP et/ou des fragments de celle-ci dans un échantillon de sérum ou de plasma par rapport à des témoins normaux est indicatrice d'un cancer du poumon.

9. Utilisation du panel de marqueurs selon la revendication 8, **caractérisé en outre en ce que** le un ou plusieurs autre marqueur est choisi dans le groupe constitué par Cyfra 21-1, CEA, CA 19-9, SCC, CA 125, NSE et/ou proGRP.

10. Utilisation du panel de marqueurs selon les revendications 8 à 9 dans l'évaluation d'un cancer du poumon, en particulier un CPNPC.


### SEQUENCE LISTING

<110> Roche Diagnostics GmbH
   F. Hoffmann-La Roche AG
<120> PACAP as a marker for cancer
<130> 25485 WO
<150> EP08019731
   <151> 2008-11-12
<160> 6
<170> PatentIn version 3.2
<210> 1
   <211> 189
   <212> PRT
   <213> Homo sapiens
<400> 1
   Met Arg Leu Ser Leu Pro Leu Leu Leu Leu Leu Leu Gly Ala Trp Ala
   1               5                   10                  15
   Ile Pro Gly Gly Leu Gly Asp Arg Ala Pro Leu Thr Ala Thr Ala Pro
               20                  25                  30
   Gln Leu Asp Asp Glu Glu Met Tyr Ser Ala His Met Pro Ala His Leu
           35                  40                  45
   Arg Cys Asp Ala Cys Arg Ala Val Ala Tyr Gln Met Trp Gln Asn Leu
       50                  55                  60
   Ala Lys Ala Glu Thr Lys Leu His Thr Ser Asn Ser Gly Gly Arg Arg
   65                  70                  75                  80
   Glu Leu Ser Glu Leu Val Tyr Thr Asp Val Leu Asp Arg Ser Cys Ser
                   85                  90                  95
   Arg Asn Trp Gln Asp Tyr Gly Val Arg Glu Val Asp Gln Val Lys Arg
               100                 105                 110
   Leu Thr Gly Pro Gly Leu Ser Glu Gly Pro Glu Pro Ser Ile Ser Val
           115                 120                 125
   Met Val Thr Gly Gly Pro Trp Pro Thr Arg Leu Ser Arg Thr Cys Leu
       130                 135                 140
   His Tyr Leu Gly Glu Phe Gly Glu Asp Gln Ile Tyr Glu Ala His Gln
   145                 150                 155                 160
   Gln Gly Arg Gly Ala Leu Glu Ala Leu Leu Cys Gly Gly Pro Gln Gly
                   165                 170                 175
   Ala Cys Ser Glu Lys Val Ser Ala Thr Arg Glu Glu Leu
               180                 185
<210> 2
   <211> 99
   <212> DNA
   <213> Artificial
<220>
   <223> Primer
<400> 2
   acgtacgtga attcattaaa gaggagaaat taactatgag aggatcgcat caccatcacc 60
   atcacattga aggccgtagg ctgtcactgc cactgctgc 99
<210> 3
   <211> 40
   <212> DNA
   <213> Artificial
<220>
   <223> Primer
<400> 3
   acgtacgtaa gctttcatta gagctcttct cttgtggctg 40
<210> 4
   <211> 14
   <212> PRT
   <213> Artificial
<220>
   <223> Polypeptide
<400> 4
   Met Arg Gly Ser His His His His His His Ile Glu Gly Arg
   1 5 10
<210> 5
   <211> 123
   <212> PRT
   <213> Homo sapiens
<400> 5
   Met Arg Leu Ser Leu Pro Leu Leu Leu Leu Leu Leu Gly Ala Trp Ala
   1               5                   10                  15
   Ile Pro Gly Gly Leu Gly Asp Arg Ala Pro Leu Thr Ala Thr Ala Pro
               20                  25                  30
   Gln Leu Asp Asp Glu Glu Met Tyr Ser Ala His Met Pro Ala His Leu
           35                  40                  45
   Arg Cys Asp Ala Cys Arg Ala Val Ala Tyr Gln Ser Leu Leu Val Pro
       50                  55                  60
   Asp Val Ala Lys Ser Gly Lys Gly Arg Asp Gln Thr Ser Tyr Leu Lys
   65                  70                  75                  80
   Leu Trp Gly Ala Ala Gly Ala Glu Arg Val Gly Leu His Gly Cys Pro
                   85                  90                  95
   Gly Pro Glu Leu Leu Pro Glu Leu Ala Gly Leu Arg Ser Ser Arg Ser
               100                 105                 110
   Gly Pro Ser Glu Thr Ser His Arg Pro Arg Thr
           115                 120
<210> 6
   <211> 61
   <212> PRT
   <213> Homo sapiens
<400> 6
   Met Val Thr Gly Gly Pro Trp Pro Thr Arg Leu Ser Arg Thr Cys Leu
   1               5                   10                  15
   His Tyr Leu Gly Glu Phe Gly Glu Asp Gln Ile Tyr Glu Ala His Gln
               20                  25                  30
   Gln Gly Arg Gly Ala Leu Glu Ala Leu Leu Cys Gly Gly Pro Gln Gly
           35                  40                  45
   Ala Cys Ser Glu Lys Val Ser Ala Thr Arg Glu Glu Leu
       50                  55                  60